Europäisches Patentamt

**European Patent Office**

**Office européen des brevets**

(19)

(11) Publication number: **0 161 703**
A1

## EUROPEAN PATENT APPLICATION

(12)

(21) Application number: 85200558.6

(22) Date of filing: 10.04.85

(51) Int. Cl.⁴: **A 61 B 3/14**, A 61 B 5/02, A 61 B 3/16, G 01 N 21/31

(30) Priority: 11.04.84 NL 8401153

(43) Date of publication of application: 21.11.85 Bulletin 85/47

(84) Designated Contracting States: DE FR GB NL

(71) Applicant: N.V. Optische Industrie "De Oude Delft", Van Miereveltlaan 9, NL-2612 XE Delft (NL)

(72) Inventor: Beckmann, Leo Heinrich Joseph Franz, Willem de Merodestraat 9, NL-2624 LC Delft (NL)
Inventor: Vlasbloem, Hugo, Burgemeester van der Lelykade 4, NL-3155 AB Maasland (NL)
Inventor: Schulte, Augustinus Victor M. C. L., Parijseplein 25, NL-3332 SL Zwijndrecht (NL)

(74) Representative: Urbanus, Henricus Maria, Ir. et al, c/o Vereenigde Octrooibureaux Nieuwe Parklaan 107, NL-2587 BP 's-Gravenhage (NL)

(54) Method and apparatus for measuring the flow of blood in the eye.

(57) Apparatus for measuring the flow of blood in the eye, in which a fluorescent substance is introduced into the bloodstream, which substance is responsive to exposure to light of a wavelength in a first range by emitting fluorescent light of a wavelength in a second range essentially separated from the first range.

The eye (13) is illuminated by a light source (3) emitting light of a wavelength in the first range. Fluorescent light is applied to a detector (22) responsive at least to light of a wavelength in the second range, which detector is adapted to produce an electrical signal proportional to the amount of fluorescent light received at each moment. The electrical signal is applied to a processing device (24).

Title: Method and apparatus for measuring the
flow of blood in the eye.

-----

The invention relates to a method of measuring the flow of blood in the eye, in which a fluorescent substance is introduced into the bloodstream, which substance is responsive to exposure to light of a wavelength in a first range for emitting fluorescent light of a wavelength in a second range essentially separated from the first range; and in which the eye is illuminated by a light source emitting light of a wavelength in the first range; as well as to an apparatus for applying the method.

Such a method, in which the fluorescent light emitted by the fluorescent substance in response to excitation by a light source is projected through suitable optical means onto a photographic film, is known per se. As the fluorescent light originates from the fluorescent substance present in the blood vessels of the eye and the intensity of the fluorescent light depends on the amount of fluorescent substance present in a blood vessel at a particular moment, an idea of the velocity of blood flow in the blood vessels of the eye can be obtained by studying a series of photographs successively taken at a suitable rate in the manner described. Conclusions as to the nature and severity of eye defects, if any, can be drawn therefrom.

The degree and velocity of the blood flow in the eye, however, can also provide valuable information on other ailments, such as hypertension and diabetes mellitus, resulting from or attended by peripheral circulatory disorders. This is important as the eye is the only part of the body where the capillary vessels are accessible for optical viewing.

A drawback inherent in the prior art technique is that photographs have to be taken which can be studied only after having been developed. Then the information scattered over a series of photographs has to be combined and only then conclusions can be drawn as to the significance of the combined information. Consequently, the prior art procedure is rather a cumbrous and lengthy affair.

Furthermore, only a limited amount of light is available for taking photographs as there is a maximum to the intensity of light to which the eye can be exposed without suffering damage, and as the blood vessels in the eye can contain only a small amount of fluorescent substance. The resultant photographs are therefore rather vague.

It is an object of the invention to eliminate the aforesaid drawbacks and, particularly, to provide a method of the type described which permits the making of real time measurements of the blood flow in the eye.

To this end, a method in accordance with the invention is characterized in that the fluorescent light is applied to a detector responsive at least to light of a wavelength in the second range, which detector is adapted to produce an electrical signal proportional to the amount of fluorescent light received at each moment, which signal is applied to a processing device.

In accordance with the invention, an apparatus for measuring the blood flow in the eye, after a fluorescent substance has been introduced into the blood stream, which substance is responsive to exposure to light of a wavelength in a first range for emitting fluorescent light of a wavelength in a second range essentially separated from the first range, which apparatus comprises a light source adapted to expose the eye via a first optical system and an exit lens to light of a wavelength in the first range, and a second optical system adapted to receive the fluorescent light, is characterized by a photodetector mounted behind the second optical system and adapted to produce an electrical signal proportional to the amount of fluorescent light received, which signal is applied to a processing device.

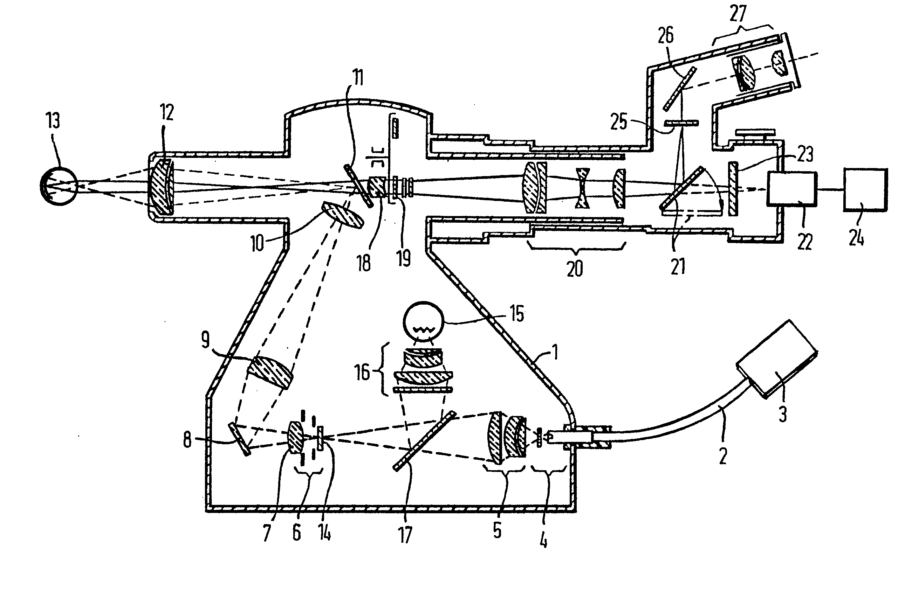

The invention will be described in greater detail hereinafter with reference to the accompanying drawing, which schematically shows in cross-sectional view an embodiment of an apparatus according to the invention.

The drawing schematically shows in cross-sectional view a prior art fundus camera modified for performing the method according to the invention. The camera shown comprises a housing 1 in which optical means are mounted for exposing an eye to light from a suitable light source, such as a laser or a Xenon lamp provided with a filter passing wavelengths in the desired range, and for receiving the fluorescent light produced in the blood vessels of the eye. The fluorescent light is produced as a fluorescent substance, such as fluorescein, is introduced into the bloodstream prior to the measurement to be made.

A light guide 2 extends into the housing 1, which light guide 2 is coupled to a light source 3 schematically shown in the drawing and mounted outside the housing 1 in the embodiment described, which light source 3 is preferably an Argon laser. The light emitted by light source 3 when operative reaches, via the light guide 2 and a coupling section 4, a condenser lens system 5 adapted to project the light onto a diaphragm means 6.

The light emanating from the diaphragm means is projected via an optical system onto the eye 13, which optical system in the embodiment described is formed by a lens 7, a mirror 8, lenses 9 and 10, a perforated mirror 11 and an exit lens 12. If desired, a filter 14 may be included in the optical system described, by means of which filter the spectrum of the light produced by light source 3 can be limited to a desired wavelength range. In the embodiment shown, a filter is mounted between the condenser lens system and the diaphragm.

Furthermore, a conventional incandescent lamp 15 may be mounted in the housing for purposes of adjustment. The light from lamp 15 is projected via a lens system 16 onto a mirror 17 reflecting the light into the direction of diaphragm 6. Mirror 17 is so constructed in known per se manner that it passes the light from the light source as incident on its rear face. Mirror 17 may also be mounted to be swung aside.

If a suitable fluorescent substance has been introduced into the bloodstream, which substance is responsive to excitation by the light from light source 3 for emitting fluorescent light of a different wavelength, this fluorescent light is projected onto a photodetector 22 via the lens 12, the aperture in the mirror 11 and a second optical system, which second optical system in the embodiment described is formed by an astigmatism compensator 18, correction lenses 19, a lens system 20 and a swing-away mirror 21. If desired, an optical bandfilter 23 may be included in the optical system, which filter only passes light of a wavelength corresponding to that of the fluorescent light. As observed earlier, the fluorescent light is in a wavelength range that does not essentially overlap the wavelength range of the light from light source 3.

The photodetector 22 converts the amount of incident light into an electrical signal to be applied to a processing device 24.

The apparatus shown further includes means for optically viewing the fluorescent light image. To this end, mirror 21 should be in the raised position shown in the drawing. Mirror 21 reflects the incident fluorescent light through an ocular plate 25, a mirror 26 and a lens system 27 to the eye of an observer.

During a measurement, however, mirror 21 is in the lowe ed position shown in broken lines in the drawing.

Photodetector 22 produces at each moment an electrical signal that is proportional to the amount of incident fluorescent light and hence to the amount of fluorescent substance present in the blood vessels of the eye. The amount of fluorescent substance present in the blood vessels of the eye depends upon the degree of blood flow in the eye, so that the electrical signal is indicative of the degree of blood flow in the eye.

An indication of the degree of blood flow in the eye is the velocity at which the blood flows through the eye. This velocity can be deduced from the electrical signal produced by the detector in a simple manner.

For example, a suitable photosensitive semiconductor device or a photomultiplier tube can be used as the photodetector.

The electrical signal can be stored as a function of time in a suitable analog memory or can be recorded on paper by means of, for example, an automatic writer. It is also possible to convert the electrical signal into a digital signal and store same in a digital memory.

Furthermore, the electrical signal can be processed in different manners, for example differentiated or integrated. The results of these operations can likewise be recorded.

Experiments have shown that a "standard" value can be established for the time required by the electrical signal to reach a certain percentage, for example 90%, of the peak value, which "standard" value can serve as a characteristic value for the blood flow in the eye, and that changes in the vascular system result in clear and reproducible deviations from the "standard" value. The peak value and the 90% value can be determined electronically in known per se manner.

Processing device 24 can be so arranged that the 90% time measured in a specific case is compared to the "standard" value and that deviations are automatically indicated by means of a numerical value displayed on a display means.

Experiments have further shown that a differentiation of the electrical signal results in variations in the velocity of the blood flow as caused by the beating of the heart becoming clearly visible, which renders it possible to check the proper operation of an apparatus according to the invention and to further check the functioning of the heart.

It is observed that the photodetector need not necessarily respond to visible light. Radiation of a wavelength outside the visible spectrum can be detected by a detector of the aforesaid type too, which permits a wider selection of a suitable combination of fluorescent substance and exciting laser.

It may be advantageous if, at the beginning of the measurement, the fluorescent substance is present in a high concentration in the blood that is about to enter the eye. This can be achieved by pinching the central retinal artery for a brief period of time at the point where it enters the eye just before the blood containing the fluorescent substance passes this point. To this end, for example, an ophthalmo-dynamometer can be utilized with means for indentation of the sclera or with a suction cup, which is actuated as soon as the first traces of the fluorescent substance have been detected by the photodetector. When the central retinal artery is released shortly thereafter, the fluorescent substance will enter the blood vessels of the eye with a sharp front.

When the ophthalmodynamometric technique described is used indeed, the command for actuating the ophthalmodynamometer can be advantageously given by processing device 24, which produces an actuating signal as soon as the signal provided by the photodetector exceeds a predetermined limit. To this end, the processing device can include a simple threshold value detector or, for example, a microcomputer which is capable of controlling the recording and presentation of the unprocessed and/or mathematically processed data and is further capable of performing the desired mathematical operations.

In the above it is assumed that the fluorescent light from the entire vascular system of the eye is detected. If desired, however, it is also possible to examine a specific part of the eye by mounting a field bounding member at a suitable location in the path between the eye and the photodetector and/or between the light source and the eye, so that only a desired part of the vascular system of the eye is exposed and/or observed.

The method according to the invention has been tested on monkeys, which have eyes similar to human ones. Use was made of a modified Zeiss fundus camera as shown in the drawing, including an Argon laser (0.2 Watts) emitting light of a wavelength of essentially 488 nanometers. As the fluorescent substance, 0.3 cc of a 10% sodium fluorescein solution was injected intravenously, which solution reached the retinal vessels after 10-14 sec. Such a solution

-10-

exhibits strong fluorescence if excited by light of a wavelength in the range of from 475 to 500 nanometers. The fluorescent light has a wavelength in the range of from 510 to 520 -nanometers. The fluorescence was measured and recorded over the entire eye, both with and without the use of the ophthalmodynamometric technique. A photocell of the BPW 35 type having a cell area of 100 square millimeters was used as the photodetector.

As stated earlier, it appeared that a "standard" value for the 90% time could be established, which value was clearly influenced by artificially widening the vessels by administering $CO_2$ or narrowing the vessels by administering $O_2$.

It is observed that instead of the 90% time, other magnitudes may be used, such as the 40% to 90% time and the like. It is also possible, after the signal produced by the detector has reached a predetermined value or after when using the ophthalmodynamometric technique, the ophthal modynamometer has been de-actuated, to integrate the output signal of the detector during a predetermined period of time and to use the thus-obtained value as an indication of the degree of blood flow in the eye.

It is further observed that the apparatus shown in the drawing is a modification of a prior art apparatus. Self-evidently, it is possible to construct an apparatus designe particularly for employing the method according to the invention, which apparatus may then have a different configuration.

It is further possible to so arrange a photodetector that it can be coupled as an accessory to a conventional fundus camera, if necessary through the intermediary of a swing-away or partly transparent mirror, so that the fundus camera can optionally be used in the conventional manner or in the manner according to the invention. In that case, however, the fundus camera should be provided with a suitable light source, which may also be in the form of an accessory, if necessary in combination with a coupling device.

***

0161703

CLAIMS:

1. A method of measuring the flow of blood in the eye, in which a fluorescent substance is introduced into the bloodstream, which substance is responsive to exposure to light of a wavelength in a first range for emitting fluorescent light of a wavelength in a second range essentially separated from said first range; and in which the eye is illuminated by a light source emitting light of a wavelength in said first range, characterized in that the fluorescent light is applied to a detector responsive at least to light of a wavelength in said second range, which detector is adapted to produce an electrical signal proportional to the amount of fluorescent light received at each moment, which signal is applied to a processing device.

2. A method according to claim 1, characterized in that the variation of said electrical signal with time is determined, and that a characteristic value for the flow of blood in the eye is determined from said variation.

3. A method according to claim 2, characterized in that said characteristic value is the time required by said electrical signal for changing from a first predetermined percentage of the ultimate peak value to a second predetermined percentage of said peak value.

4. A method according to claim 3, characterized in that said first percentage is 0% and said second percentage is 90%.

5. A method according to claim 2, characterized in that the variation of said electrical signal with time during a predetermined period of time is integrated, and that the result of the integration is used as the characteristic value for the flow of blood in the eye.

6. A method according to claim 2, characterized in that the variation of said electrical signal with time is differentiated.

7. A method according to any one of the preceding claims, characterized in that field bounding is employed so that only fluorescent light from a part of the blood vessels of the eye is incident on the detector.

8. A method according to any one of the preceding claims, in which the central retinal artery is pinched to during a brief period of time by means of an ophthalmodynamometric technique as soon as said electrical signal has reached a predetermined value.

9. An apparatus for measuring the flow of blood in the eye, after a fluorescent substance has been introduced into the bloodstream, which substance is responsive to exposure to light of a wavelength in a first range for emitting fluorescent light of a wavelength in a second range essentially separated from said first range, which apparatus comprises a light source adapted to expose the eye via a first optical system and an exit lens to light of a wavelength in said first range, and a second optical system adapted to receive the fluorescent light, characterized by a photodetector mounted behind said second optical system and adapted to produce an electrical signal proportional to the amount of fluorescent light received, which signal is applied to a processing device.

10. An apparatus according to claim 9, characterized in that said photodetector is a semiconductor device responsive to light of wavelengths in the desired range.

11. An apparatus according to claim 9, characterized in that said photodetector is a photomultiplier tube.

12. An apparatus according to claim 9, characterized in that said processing device includes a memory means recording the variation of said electrical signal with time.

13. An apparatus according to claim 12, characterized in that said processing device is adapted to determine a value characteristic of the flow of blood in the eye from the variation of said electrical signal with time.

14. An apparatus according to claim 12, characterized in that said processing device determines the peak value of said electrical signal as well as the period of time required by said electrical signal for changing from a value corresponding to a first percentage of said peak value to a value corresponding to a second percentage of said peak value, and presents said period of time as said characteristic value.

15. An apparatus according to claim 12, characterized in that said processing device includes a differentiating circuit adapted to differentiate said electrical signal.

16. An apparatus according to claim 12, characterized in that said processing device includes an integrating circuit adapted to integrate said electrical signal during a predetermined period of time.

17. An apparatus according to claim 9, characterized by a threshold value detector included in said processing device, which detector produces a signal as soon as said electrical signal has reached a predetermined value.

18. An apparatus according to claim 9, characterized in that said processing device includes a microcomputer.

19. A photoresponsive detector responsive to radiation in a given range of wavelengths for producing an electrical signal, which detector can be connected to a processing device, characterized in that at least said photoresponsive detector is mounted in a housing adapted to be coupled to a conventional fundus camera in such a manner that fluorescent light from the eye is received by said detector.

20. An apparatus according to any one of preceding claims 9-18, characterized in that said light source is a laser adapted to emit light of a wavelength in said first range.

21. An apparatus according to any one of preceding claims 9-19, characterized in that said first optical system includes a filter passing only light of a wavelength in said first range.

22. An apparatus according to any one of claims 9-19, characterized in that said light source is a Xenon lamp and that said first optical system includes a filter passing only light of a wavelength in said first range.

23. An apparatus according to any one of claims 9-22, characterized in that a field bounding means is mounted in the total light path between light source and exit lens and between exit lens and eye, respectively, so that a desired part of the eye can be examined.

| | European.Patent Office | **PARTIAL EUROPEAN SEARCH REPORT** which under Rule 45 of the European Patent Convention shall be considered, for the purposes of subsequent proceedings, as the European search report | Application number EP 85 20 0558 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A- 3 117 699 (METRICON Ltd.)<br><br>* Abstract; page 12, line 8 - page 13, line 9; page 15, line 10 - page 18, line 20; page 19, lines 4-13; page 20, line 13 - page 21, line 17; page 32, line 3 - page 34, line 8; figures 1,2,8,9 *<br><br>-- | 1,5,7, 9,10, 16,18, 19,21, 23 | A 61 B 3/14<br>A 61 B 5/02<br>A 61 B 3/16<br>G 01 N 21/31 |
| X | EP-A- 0 059 032 (V.B. ELINGS)<br><br>* Abstract; page 5, lines 1-27; page 9, lines 1-21; page 11, lines 4-18; page 13, lines 11-16; page 18, line 23 - page 19, line 9; page 20, lines 1-6; figures 1-5 *<br><br>-- | 1,2,9-13,20, 21 | |
| X | PHYSICS IN MEDICINE AND BIOLOGY, vol.24, no.3, May 1979, pages 489-504; London, GB<br>R.A. WEALE: "Physics and ophthalmology." ./. | | **TECHNICAL FIELDS SEARCHED (Int. Cl.4)**<br><br>A 61 B<br>A 61 F |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 9-23
Claims searched incompletely: 1-8
Claims not searched:
Reason for the limitation of the search: Method for treatment of the human or animal body by surgery or therapy (See art. 52(4) of the European Patent Convention).

| Place of search | Date of completion of the search | Examiner |
|---|---|---|
| The Hague | 23-07-1985 | RIEB |

EPO Form 1505.1.03.82

# European Patent Office

## PARTIAL EUROPEAN SEARCH REPORT

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| | * Pages 493-496, paragraph 3: "Fluorescein angiography" * | 1,9, 21 | |
| A | | 19,22 | |
| | -- | | |
| A | BRITISH KINEMATOGRAPHY SOUND AND TELEVISION, vol.52, no.1, January 1970, pages 14-16; London, GB C.J. BULPITT et al.: "Retinal cine angiography." | | |
| | * Whole article * | 1,2, 11-13, 19,22 | |
| | -- | | **TECHNICAL FIELDS SEARCHED (Int. Cl.4)** |
| A | DE-B- 1 078 735 (F. SCHWARZER) | | |
| | * Column 1, line 20 - column 2, line 47; figures 1,2 * | 8 | |
| | -- | | |
| A | US-A- 3 835 836 (Y.C. KANTER et al) | | |
| | * Abstract; column 2, line 60 - column 3, line 12; column 4, line 31 - column 5, line 31; column 6, lines 47-56; figures 1,2 * | 8 | |
| | -- | | |
| A | PROCEEDINGS OF THE 23RD ANNUAL CONFERENCE ON ENGINEERING IN MEDICINE AND BIOLOGY, November 15-19, 1970, Washington D.C., page 31, article 4.7; IEEE; New York, US N.A. PALLEY et al.: "A computerized system for routine cardiac output measurements." | | |
| | * Whole article * | 2,3, 5,12, 14, 16-18 | |
| | -- | | |
| A | US-A- 3 651 318 (J.A. CZEKAJEWSKI) | | |
| | * Abstract; column 3, lines 34-57; | 2,3, | |

./.

EPO Form 1505.3  06.78

## PARTIAL EUROPEAN SEARCH REPORT

**European Patent Office**

Application number

EP 85 20 0558

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| | column 6, lines 20-25; figures 1-3 * | 5, 12-14, 16,17 | |
| | -- | | |
| P,X | NL-A- 83 01 049 (A.V.M.C.L. SCHULTE) | | |
| | * Page 1, lines 1-34; page 2, line 23 - page 3, line 4; page 4, line 3 - page 5, line 20; figures 1,2 * | 1,9, 20-22 | |
| | ------- | | **TECHNICAL FIELDS SEARCHED (Int. Cl.4)** |

EPO Form 1505.3   06.78